# EUROPÄISCHE PATENTSCHRIFT

(11) **EP 2 417 988 B1**
(45) Veröffentlichungstag und Bekanntmachung des Hinweises auf die Patenterteilung: **10.10.2018**
(21) Anmeldenummer: 11004996.2
(22) Anmeldetag: 20.06.2011
(51) Int. Cl.: A61L 24/06, A61L 27/16, A61L 24/00, A61L 27/50

(54) **PASTENFÖRMIGER KNOCHENZEMENT**
BONE CEMENT IN PASTE FORM
CIMENT OSSEUX PÂTEUX

(30) Priorität: 22.06.2010 DE 102010024653
(43) Veröffentlichungstag der Anmeldung: 15.02.2012
(73) Patentinhaber: Heraeus Medical GmbH, 61273 Wehrheim (DE)
(72) Erfinder: VOGT, Sebastian, 99092 Erfurt (DE); BÜCHNER, Hubert, 90491 Nürnberg (DE)
(74) Vertreter: Heraeus IP

(56) Entgegenhaltungen:
- EP-A2- 2 052 748
- DE-A1- 19 941 738
- DE-A1-102007 050 763

## Beschreibung

Die vorliegende Erfindung betrifft einen Kit zur Herstellung von Knochenzement und Verwendungen dieses Kits.

Konventionelle Polymethylmethacrylat-Knochenzemente (PMMA-Knochenzemente) sind seit Jahrzehnten bekannt und gehen auf die grundlegenden Arbeiten von Sir Charnley zurück (Charnley, J.: Anchorage of the femoral head prosthesis of the shaft of the femur. J. Bone Joint Surg. 42 (1960) 28-30). Der Grundaufbau der PMMA-Knochenzemente ist seitdem prinzipiell gleich geblieben. PMMA-Knochenzemente bestehen aus einer flüssigen Monomerkomponente und einer Pulverkomponente. Die Monomerkomponente enthält im Allgemeinen (i) das Monomer Methylmethacrylat und (ii) einen darin gelösten Aktivator (zum Beispiel N,N-Dimethyl-p-toluidin). Die Pulverkomponente umfasst (i) ein oder mehrere Polymere, die auf Basis von Methylmethacrylat und Comonomeren, wie Styren, Methylacrylat oder ähnlichen Monomeren, durch Polymerisation, vorzugsweise Suspensionspolymerisation, hergestellt sind, (ii) einen Röntgenopaker und (iii) einen Initiator (zum Beispiel) Dibenzoylperoxid. Beim Vermischen der Pulverkomponente mit der Monomerkomponente entsteht durch Quellung der Polymere der Pulverkomponente im Methylmethacrylat der Monomerkomponente ein plastisch verformbarer Teig. Gleichzeitig reagiert der Aktivator N,N-Dimethyl-p-toluidin mit Dibenzoylperoxid, das unter Bildung von Radikalen zerfällt. Die gebildeten Radikale initiieren die radikalische Polymerisation des Methylmethacrylates. Mit fortschreitender Polymerisation des Methylmethacrylates erhöht sich die Viskosität des Zementteigs bis der Zementteig erstarrt und damit ausgehärtet ist.

Der wesentliche Nachteil der bisherigen PMMA-Knochenzemente für den medizinischen Anwender besteht darin, dass der Anwender die flüssige Monomerkomponente mit der Pulverkomponente unmittelbar vor der Zementapplikation in einem Mischsystem oder in Tiegeln vermischen muss. Dabei können leicht Mischungsfehler auftreten, die die Zementqualität negativ beeinflussen. Ferner muss das Vermischen der Komponenten zügig erfolgen. Dabei kommt es darauf an, dass das gesamte Zementpulver ohne Klumpenbildung mit der Monomerkomponente vermischt wird und das beim Vermischungsvorgang der Eintrag von Luftblasen vermieden wird. Bei der Verwendung von Vakuummischsystemen wird im Gegensatz zur Handanmischung die Bildung von Luftblasen im Zementteig weitgehend verhindert. Beispiele für Mischsysteme sind in den Schriften US 4015945, EP 0674888 und JP 2003181270 offenbart. Vakuummischsysteme machen jedoch eine zusätzliche Vakuumpumpe erforderlich und sind daher relativ kostenintensiv. Nach der Vermischung der Monomerkomponente mit der Pulverkomponente muss ferner je nach Zementtyp eine gewisse Zeit gewartet werden, bis der Zementteig klebfrei ist und appliziert werden kann. Aufgrund der vielen Fehlermöglichkeiten beim Anmischen von konventionellen PMMA-Knochenzementen wird zudem entsprechend geschultes Personal benötigt. Die entsprechenden Schulungen sind mit einem erheblichen Kostenaufwand verbunden. Weiterhin kommt es bei der Vermischung der flüssigen Monomerkomponente mit der Pulverkomponente zu einer Belastung der Anwender durch Monomerdämpfe und durch Freisetzung von pulverförmigen Zementpartikeln.

Als Alternative zu den konventionellen Pulver-Flüssigkeits-Polymethylmethacrylat-knochenzementen wurden pastenförmige Polymethylmethacrylatknochenzemente in den Offenlegungsschriften DE 102007052116 und DE 102007050763 beschrieben. Dabei werden die Knochenzemente als vorgemischte, lagerstabile Pasten dem Anwender zur Verfügung gestellt. Im Fall von pastenförmigen Zweikomponentenzementen sind der Initiator und der Beschleuniger in jeweils einer Zementpaste separat gelöst. Bei Vermischung der beiden Pasten reagiert der Beschleuniger mit dem Initiator, wobei Radikale gebildet werden, die die radikalische Polymerisation des Monomers in der Paste initiieren. Dadurch wird die Aushärtung der Zementpaste gestartet. Bei pastenförmigen Einkomponentensystemen kann die Polymerisation mit thermisch zerfallenden Initiatoren durch Einwirkung von magnetischen oder elektromagnetischen Feldern auf in der Paste enthaltende ferromagnetische Partikel oder superparamagnetische Partikel ausgelöst werden.

Initiatorsysteme für die radikalische Polymerisation von Methacrylatmonomeren und anderen radikalisch polymerisierbaren Monomeren sind seit langem bekannt.

So wird in der DE 69621500 eine Kombination von Peroxiden und Metallverbindungen offenbart. Dabei kommt eine Kombination aus Cumenhydroperoxid, einer Metallverbindung und Thioharnstoff zu Anwendung. Eine ähnliche Kombination aus Thioharnstoff und einem Hydroperoxid wird in der EP 1479364 vorgeschlagen. In der DE 19501933 sind dagegen Mischungen aus Hydroperoxiden und Sikkativen offen gelegt. Ein neues interessantes System basierend auf Hydroperoxiden, Acylthioharnstoffen und Kupfersalzen wird in der EP 1754465 dargestellt. Der Vorteil von solchen Initiatorsystemen besteht in ihrer hohen thermischen Stabilität. Hydroperoxide sind jedoch reizende Verbindungen und daher nur bedingt geeignet zur Initiierung von PMMA-Knochenzmenten, die direkten Kontakt zu vitalem Knochengewebe haben.

Das bei den bisherigen aus einer Pulverkomponente und einer Monomerflüssigkeit bestehenden PMMA-Knochenzementen eingesetzte Initiatorsystem Dibenzoylperoxid und N,N-Dimethyl-p-toluidin hat sich prinzipiell bewährt (K.-D. Kühn: Knochenzemente für die Endoprothetik: ein aktueller Vergleich der physikalischen und chemischen Eigenschaften handelsüblicher PMMA-Zemente. Springer-Verlag Berlin Heidelberg New York, 2001). Das Dibenzoylperoxid liegt dabei als Feststoff im Zementpulver vor und das N,N-Dimethyl-p-toluidin ist in der Monomerkomponente gelöst.

Eigene Versuche mit Zementpasten unter Verwendung des Initiatorsystems Dibenzoylperoxid/N,N-Dimethyl-p-toluidin zeigten allerdings, dass Pasten mit N,N-Dimethyl-p-toluidin eine ausgeprägte Tendenz zur Spontanpolymerisation besitzen. Weiterhin wird der bei konventionellen Pulver/Flüssigkeits-Polymethylmethacrylat-Knochenzementen bewährte Beschleuniger N,N-Dimethyl-p-toluidin toxikologisch kritisch diskutiert.

Neben diesem Redoxsystem wurden auch Initiatorsysteme beschrieben, die auf der Verwendung von Barbituraten beruhen.

In der DE 1495520 wurde ein Verfahren zur Polymerisation von Vinylverbindungen und Polyestern beschrieben. Dabei werden Barbitursäurederivate, Halogenid-Ionendonatoren und Kupferverbindungen im Monomer oder Monomergemisch gelöst. Die Kombination Barbitursäurederivat, Halogenid-Ionendonator und Kupferverbindung initiiert dabei die Polymerisation. Es können dazu auch organische Peroxide oder Wasserstoffperoxid zugesetzt werden. Eigene Versuche dazu zeigten, dass, entgegen der Annahme in der DE 1495520, wonach Luft oder Peroxide zur Auslösung der Polymerisation durch Barbiturat in Gegenwart von Kupfer-Ionen und ChloridIonen notwendig sind, auch ohne Luftsauerstoff oder Peroxide eine Initiierung möglich ist. Das bedeutet, dass offensichtlich das Barbiturat selbst als Initiator wirkt.

In der US 2003/0195273 wird eine härtbare Komposition für Dentalanwendungen vorgeschlagen, die neben ungesättigten Monomeren, Wasser, ein Redoxinitiatorsystem und Ammoniumsalze enthält. Dabei wird auch das aus der DE 1495520 bekannte Initiatorsystem auf Basis von Barbituraten erwähnt.
Ein Primer auf der Basis von einem mit Wasser mischbaren Monomer, einem Kupfersalz, einem Chlorid-Ionendonator und Thiobarbituraten sowie Barbituraten wurde in der GB 2256875 offengelegt. Es gibt keine Hinweise auf die Verwendung von Kupfer(II)-hydroxid oder von basischem Kupfercarbonat.
In der WO 2007/1400440 wird ein sehr interessantes System beschrieben, bei dem ein in nicht aciden Monomeren unlösliches Alkalisalz oder Erdalkalisalz von Barbitursäure in Pasten eingesetzt wird. Durch Einwirkung von aciden Monomeren auf diese Barbituratsalze werden die Barbiturate durch einen Kationenaustausch freigesetzt. Die freigesetzten Barbiturate reagieren mit in der Pasten vorliegenden gelösten Kupfer-Ionen in Gegenwart von Halogenidionen-Donatoren und initiieren dadurch die radikalische Polymerisation.
Ein etwas komplexeres System wird in der DE 102007050763 beschrieben. Bei diesem System sind Erdalkalisalze von Barbituraten und basische Kupfersalze in einer Paste enthalten. Diese beiden Salze sind im Methacrylat-Monomeren unlöslich. In einer zweiten Paste ist eine schwache organische Säure, wie 2-Ethylhexansäure, enthalten. Daneben ist noch ein Chlorid-Ionendonator in den Pasten vorhanden. Bei Vermischung der beiden Pasten wird durch die schwache organische Säure synchron sowohl das Barbiturat in die lösliche Säureform als auch das Kupfer in ein lösliches Kupfersalz überführt. Der Vorteil dieses Systems ist insbesondere bei Pasten mit mehrfachfunktionellen Monomeren, dass durch vorgelagerte Diffusions- und lonenaustauschprozesse eine Erhöhung der Verarbeitungszeit möglich ist, die sonst bei der Verwendung von mehrfach funktionellen Monomeren nur sehr kurz ist und üblicherweise im Sekundenbereich liegt.

In der DE 102007050762 [EP-A-2052748] wird ein weiteres System zur Herstellung von Knochenzement offenbart. Die darin beschriebene Erfindung basiert auf der Idee, zwei Pasten vorzusehen, die jeweils ein Methacrylatmonomer enthalten, in dem ein darin lösliches Polymer gelöst und ein darin nicht Iösliches. Polymer suspendiert ist. Dadurch gelingt es, eine teigartige Masse herzustellen, die durch das gelöste Polymer einen hohen inneren Zusammenhalt zeigt. Zur Aushärtung des Methacrylatmonomers ist in einer der Pasten ein radikalischer Initiator, wie zum Beispiel ein Barbitursäurederivat, und in der anderen Paste ein Akzelerator, wie zum Beispiel ein organisches Kupfer(II)-salz, enthalten. Nach dem Vermischen der beiden Pasten wird durch Aktivierung des Initiators die Polymerisation des Methacrylatmonomers unter Ausbildung von Knochenzement mit einer hohen 4-Punkt-Biegefestigkeit und einem hohen Biegemodul gestartet.

Es zeigte sich in Versuchen, dass bei der Verwendung von pastenförmigen ZweikomponentenZementen, die im Methacrylat-Monomer unlösliche Füllstoffe, wie zum Beispiel Polymerpartikel, enthalten, eine geringe initiale Festigkeit und damit einhergehend eine ausgeprägte Tendenz zur Nachhärtung auftritt. Dieser Effekt ist auf Monomer zurückzuführen, das in den unlöslichen Füllstoffen enthalten ist. Bei der Aushärtung erfolgt im Wesentlichen die Polymerisation des Monomers außerhalb der unlöslichen Polymerpartikel. Danach diffundiert das restliche Monomer zusammen mit dem gelösten Initiator oder dem gelösten Beschleuniger aus den Polymerpartikeln und wird nachpolymerisiert. In Versuchen zeigte sich, dass der Nachhärtungseffekt wesentlich durch die Paste verursacht wird, in der der Beschleuniger gelöst ist. Es wäre daher vorteilhaft, möglichst beide Pasten des Zweikomponentenknochenzementes mit Initiatoren zu versehen. Dabei gibt es jedoch den technischen Widerspruch, dass der Beschleuniger beide Initiatoren zur Radikalbildung bringen muss und andererseits keine vorzeitige Polymerisation während der Lagerung der Zementpasten auslösen darf.

Der vorliegenden Erfindung lag somit die Aufgabe zugrunde, einen Kit auf der Basis zweier Pasten bereitzustellen, der zur Herstellung von Knochenzement mit einer hohen initialen Festigkeit und damit einer geringen Nachhärtung vorgesehen ist.

Diese Aufgabe wird gelöst durch einen Kit, der eine Paste A und eine Paste B aufweist, wobei
(a) Paste A
   (a1) ein polymerisierbares Monomer mit einem pH-Wert in Wasser im Bereich von 5 - 9,
   (a2) einen in (a1) unlöslichen Füllstoff und
   (a3) ein Barbitursäurederivat, das aus der Gruppe ausgewählt ist, die aus 1,5-disubstituierten Barbituraten, 1,3,5-trisubtituierten Barbituraten und 1,3,5-tetrasubtituierten Barbituraten besteht, enthält, und
(b) Paste B
   (b1) ein polymerisierbares Monomer mit einem pH-Wert in Wasser im Bereich von 5 - 9,
   (b2) einen in (b1) unlöslichen Füllstoff,
   (b3) ein in (b1) lösliches Peroxid,
   (b4) eine in (b1) unlösliche Schwermetallverbindung, die aus der Gruppe ausgewählt ist, die aus Schwermetallsalzen und Schwermetallkomplexen besteht, enthält,
wobei in wenigstens einer der Pasten A und B ein Halogenidsalz enthalten ist.

Die Erfindung basiert auf der Idee, einen im Methacrylat-Monomer gelösten peroxidischen Initiator in einer Paste zu verwenden und in dieser Paste zusätzlich ein in diesem Methacrylat-Monomer unlösliches basisches Schwermetallsalz zu suspendieren. Es zeigte sich überraschend, dass im Methacrylat-Monomer suspendierte unlösliche Schwermetallsalze, wie Kupfer(II)-hydroxid, das im Methacrylat-Monomer gelöste Peroxid nicht zersetzen. Somit kommt es in dieser Paste nicht zu einer unerwünschten Spontanpolymerisation. Die Erfindung basiert weiterhin auf der Idee, eine zweite Paste zu verwenden, die ein in Methylmethacrylat lösliches Barbiturat enthält. Bei Vermischung der beiden Pasten reagiert das lösliche Barbiturat auf Grund seiner Acidität mit dem basischen Schwermetallsalz. Überraschend zeigte sich, dass dadurch die Polymerisationsreaktion in Gegenwart eines löslichen Halogenidionen-Donators initiiert werden kann. Durch Einwirkung des Barbiturats auf das basische Schwermetallsalz werden die Schwermetallionen offensichtlich in eine lösliche Salzform überführt, welche durch Einwirkung auf die Barbitursäure und das Peroxid die Polymerisation des Methacrylatmonomeren initiiert. Die Verwendung von aromatischen Aminen als Beschleuniger ist dabei nicht mehr notwendig.

Folglich sind in den pastenförmigen Komponenten des Kits zur Herstellung von Knochenzement jeweils ein Initiator (Barbitursäurederivat bzw. Peroxid) und in wenigstens einer dieser pastenförmigen Komponenten auch ein Beschleuniger (unlösliche Schwermetallverbindung) enthalten. Überraschenderweise kommt es dabei jedoch vor der gezielten Vermischung der beiden Pasten nicht zu einer unerwünschten vorzeitigen Polymerisation. Dadurch wird der technische Widerspruch überwunden, dass der Beschleuniger einerseits beide Initiatoren zur Radikalbildung bringen muss, andererseits aber keine vorzeitige Polymerisation während der Lagerung der Zementpasten auslösen darf. Durch Verwendung des erfindungsgemäßen Initiatorsystems und das Vorsehen eines Polymerisationsinitiators in jeder der Kitkomponenten kann wirksam eine hohe initiale Festigkeit des Knochenzements erreicht und eine ausgeprägte Nachhärtung des Knochenzements verhindert werden.

Erfindungsgemäß wird unter Kit ein System aus wenigstens zwei Komponenten verstanden. Obwohl im Folgenden von zwei Komponenten die Rede ist, kann der Kit falls notwendig auch mehr als zwei Komponenten, beispielsweise drei, vier, fünf oder mehr als fünf Komponenten enthalten. Die einzelnen Kitkomponenten liegen vorzugsweise getrennt voneinander verpackt vor, so dass die Inhaltsstoffe der einen Kitkomponente mit den Inhaltsstoffen einer weiteren Kitkomponente nicht in Kontakt stehen. Beispielsweise ist es möglich, die jeweiligen Kitkomponenten getrennt voneinander zu verpacken und zusammen in einem Vorratsbehälter aufzubewahren.

Der Kit weist erfindungsgemäß wenigstens eine Paste A und eine Paste B auf.

Paste A enthält wenigstens ein polymerisierbares Monomer (a1) mit einem pH-Wert in Wasser im Bereich von 5 - 9.

Das polymerisierbare Monomer (a1) ist bei einer Temperatur von 25°C und einem Druck von 1013 hPa vorzugsweise flüssig.

Das polymerisierbare Monomer (a1) ist vorzugsweise ein Methacrylsäureester. Vorzugsweise handelt es sich bei dem Methacrylsäureester (a1) um einen monofunktionellen Methacrylsäureester. Dieser ist vorzugsweise hydrophob. Durch die Verwendung von hydrophoben monofunktionellen Methacrylsäureestern (a1) kann eine spätere Volumenvergrößerung des Knochenzements infolge von Wasseraufnahme und damit eine Beschädigung des Knochens verhindert werden. Gemäß einer bevorzugten Ausführungsform ist der monofunktionelle Methacrylsäureester hydrophob, wenn er neben der Estergruppe keine weiteren polaren Gruppen enthält. Vorzugsweise weist der monofunktionelle, hydrophobe Methacrylsäureester keine Carboxylgruppen, Hydroxylgruppen, Amidgruppen, Sulfonsäuregruppen, Sulfatgruppen, Phosphatgruppen oder Phosphonatgruppen auf.

Bei den Estern handelt es sich vorzugsweise um Alkylester. Unter Alkylester fallen erfindungsgemäß auch Cycloalkylester. Die Alkylester sind gemäß einer bevorzugten Ausführungsform Ester von Methacrylsäure mit Alkoholen, die 1-20 Kohlenstoffatome, mehr bevorzugt 1 - 10 Kohlenstoffatome, noch mehr bevorzugt 1 - 6 Kohlenstoffatome und ganz besonders bevorzugt 1 - 4 Kohlenstoffatome aufweisen. Die Alkohole können substituiert oder nicht substituiert sein und sind vorzugsweise nicht substituiert. Die Alkohole können ferner gesättigt oder ungesättigt sein und sind vorzugsweise gesättigt.

Gemäß einer besonders bevorzugten Ausführungsform handelt es sich bei dem polymerisierbaren Monomer (a1) um Methacrylsäuremethylester und Methacrylsäureethylester.

Gemäß einer weiteren bevorzugten Ausführungsform handelt es sich bei dem polymerisierbaren Monomer (a1) nicht um einen von Bisphenol A abgeleiteten Methacrylsäureester.

Das erfindungsgemäß eingesetzte polymerisierbare Monomer (a1) weist vorzugsweise eine Molmasse von weniger als 1000 g/mol auf. Davon umfasst sind auch polymerisierbare Monomere, die Bestandteil einer Monomermischung sind, wobei wenigstens eines der polymerisierbaren Monomere der Monomermischung eine definierte Struktur mit einer Molmasse von weniger als 1000 g/mol aufweist.

Das polymerisierbare Monomer (a1) zeichnet sich dadurch aus, dass eine wässrige Lösung des polymerisierbaren Monomers (a1) einen pH-Wert im Bereich von 5 - 9, vorzugsweise im Bereich von 5,5 - 8,5, noch mehr bevorzugt im Bereich von 6 - 8 und ganz besonders bevorzugt im Bereich von 6,5 - 7,5 aufweist.

Paste A enthält vorzugsweise 15 - 85 Gewichtsprozent, mehr bevorzugt 20 - 70 Gewichtsprozent, noch mehr bevorzugt 25 - 60 Gewichtsprozent und besonders bevorzugt 25 - 50 Gewichtsprozent wenigstens eines polymerisierbaren Monomers (a1), bezogen auf das Gesamtgewicht der in Paste A enthaltenen Bestandteile. Demnach kann Paste A ein oder mehrere strukturell verschiedene polymerisierbare Monomere (a1) enthalten.

Paste A enthält ferner wenigstens einen in (a1) unlöslichen Füllstoff (a2).

Bei dem Füllstoff (a2) handelt es sich um einen bei Raumtemperatur festen Stoff, der in der Lage ist, die Viskosität der aus den übrigen in Paste A enthaltenen Bestandteilen zusammengesetzten Mischung zu erhöhen. Der Füllstoff (a2) soll biokompatibel sein.

Gemäß einer bevorzugten Ausführungsform ist der Füllstoff (a2) aus Polymeren, anorganischen Salzen, anorganischen Oxiden, Metallen und Metalllegierungen ausgewählt.

Der Füllstoff (a2) ist vorzugsweise partikulär. Gemäß einer besonders bevorzugten Ausführungsform weist der Füllstoff (a2) eine durchschnittliche Teilchengröße im Bereich von 10 nm - 100 µm und besonders bevorzugt im Bereich von 100 nm - 10 µm auf. Unter durchschnittlicher Teilchengröße wird vorliegend ein Größenbereich verstanden, der von wenigstens 90 Prozent der Teilchen eingenommen wird.

Im Rahmen der Erfindung werden unter dem Begriff Polymere sowohl Homopolymere als auch Copolymere zusammengefasst.

Bei dem als Füllstoff verwendbaren Polymer handelt es sich vorzugsweise um ein Polymer mit einer gewichtsmittleren Molmasse von wenigstens 150.000 g/mol. Die Angabe der Molmasse bezieht sich hierin auf die viskosimetrisch bestimmte Molmasse. Beispielsweise kann es sich bei dem Polymer um ein Polymer oder Copolymer eines Methacrylsäureesters handeln. Gemäß einer besonders bevorzugten Ausführungsform ist das wenigstens eine Polymer aus der Gruppe ausgewählt, die aus Polymethacrylsäuremethylester (PMMA), Polymethacrylsäureethylester (PMAE), Polymethacrylsäurepropylester (PMAP), Polymethacrylsäureisopropylester, Poly(methylmethacrylat-co-methylacrylat) und Poly(styren-co-methylmethacrylat) besteht. Das Polymer kann jedoch ebenso aus der Gruppe ausgewählt sein, die aus Polyethylen, Polypropylen oder Polybutadien besteht. Das Polymer kann zudem vernetzt oder unvernetzt sein.

Das als Füllstoff (a2) verwendbare anorganische Salz kann ein im polymeriserbaren Monomer lösliches oder unlösliches Salz sein. Vorzugsweise handelt es sich bei dem anorganischen Salz um ein Salz eines Elements, das aus der 2. Hauptgruppe des Periodensystems der Elemente ausgewählt wird. Gemäß einer bevorzugten Ausführungsform ist das anorganische Salz ein Salz von Calcium, Strontium oder Barium. Gemäß einer besonders bevorzugten Ausführungsform ist das anorganische Salz Calciumsulfat, Bariumsulfat oder Calciumcarbonat.

Bei dem als Füllstoff (a2) verwendbaren anorganischen Oxid kann es sich vorzugsweise um ein Metalloxid handeln. Gemäß einer bevorzugten Ausführungsform ist das anorganische Oxid ein Oxid der Übergangsmetalle. Gemäß einer besonders bevorzugten Ausführungsform handelt es sich bei dem anorganischen Oxid um Titandioxid oder Zirkoniumdioxid.

Bei dem als Füllstoff (a2) verwendbaren Metall kann es sich beispielsweise um ein Übergangsmetall handeln. Gemäß einer bevorzugten Ausführungsform ist das Metall Tantal oder Wolfram.

Die als Füllstoff (a2) verwendbare Metalllegierung ist eine Legierung von wenigstens zwei Metallen. Vorzugsweise enthält die Legierung wenigstens ein Übergangsmetall. Gemäß einer besonders bevorzugten Ausführungsform weist die Legierung wenigstens Tantal oder Wolfram auf. Bei der Legierung kann es sich auch um eine Legierung aus Tantal und Wolfram handeln.

Der Füllstoff (a2) ist in dem polymerisierbaren Monomer (a1) mit einem pH-Wert im Bereich von 5 - 9 (a1) unlöslich. Erfindungsgemäß ist der Füllstoff (a2) in dem polymerisierbaren Monomer mit einem pH-Wert im Bereich von 5 - 9 (a1) unlöslich, wenn der Füllstoff (a2) bei einer Temperatur von 25°C eine Löslichkeit im polymerisierbaren Monomer (a1) von weniger als 50 g/l, vorzugsweise weniger als 25 g/l, mehr bevorzugt weniger als 10 g/l und noch mehr bevorzugt weniger als 5 g/l aufweist.

Der Anteil des wenigstens einen Füllstoffs (a2) beträgt vorzugsweise weniger als 85 Gewichtsprozent, mehr bevorzugt weniger als 80 Gewichtsprozent und noch mehr bevorzugt weniger als 75 Gewichtsprozent, bezogen auf das Gesamtgewicht der in Paste A enthaltenen Bestandteile. Vorzugsweise enthält Paste A 15 - 85 Gewichtsprozent, mehr bevorzugt 15 - 80 Gewichtsprozent und noch mehr bevorzugt 20 - 75 Gewichtsprozent an dem wenigstens einen Füllstoff (a2), bezogen auf das Gesamtgewicht der in Paste A enthaltenen Bestandteile.

Paste A enthält ferner wenigstens ein Barbitursäurederivat (a3). Dieses Barbitursäurederivat (a3) ist aus der Gruppe ausgewählt, die aus 1,5-disubstituierten Barbituraten, 1,3,5-trisubtituierten Barbituraten und 1,3,5-tetrasubtituierten Barbituraten besteht.

Gemäß einer bevorzugten Ausführungsform ist das Barbitursäurederivat (a3) in dem polymerisierbaren Monomer (a1) löslich. Das Barbitursäurederivat (a3) ist in dem polymerisierbaren Monomer (a1) löslich, wenn sich das Barbitursäurederivat (a3) in dem polymerisierbaren Monomer (a1) zu wenigstens 1 g/l, vorzugsweise zu wenigstens 3 g/l, noch mehr bevorzugt zu wenigstens 5 g/l und ganz besonders bevorzugt zu wenigstens 10 g/l bei einer Temperatur von 25°C löst.

Die Art der Substituenten an der Barbitursäure ist nicht weiter eingeschränkt. Bei den Substituenten kann es sich beispielsweise um aliphatische oder aromatische Substituenten handeln. Hierbei können alkylische, cycloalkylische, allylische oder arylische Substituenten bevorzugt sein. Die Substituenten können auch Heteroatome tragen. Insbesondere kann es sich bei den Substituenten um Thiosubstituenten handeln. Demnach können 1,5-disubstituierte Thiobarbiturate oder 1,3,5-trisubstituierte Thiobarbiturate bevorzugt sein.

Gemäß einer bevorzugten Ausführungsform weisen die Substituenten jeweils eine Länge von 1 - 10 Kohlenstoffatomen, mehr bevorzugt eine Länge von 1 - 8 Kohlenstoffatomen und ganz besonders bevorzugt eine Länge im Bereich von 2 - 7 Kohlenstoffatomen auf. Erfindungsgemäß bevorzugt sind Barbiturate, die an Position 1 und an Position 5 jeweils einen Substituenten, an den Positionen 1, 3 und 5 jeweils einen Substituenten oder an den Positionen 1 und 3 jeweils einen Substituenten und an Position 5 zwei Substituenten tragen.

Gemäß einer weiteren bevorzugten Ausführungsform ist das Barbitursäure-Derivat ein 1,5-disubstituiertes Barbiturat oder ein 1,3,5-trisubtituiertes Barbiturat. 1,3,5-tetrasubtituierte Barbiturate können ebenfalls verwendet werden, weisen jedoch die Eigenschaft auf, die Blut-Hirn-Schranke überwinden zu können und daher pharmakologisch aktiv zu sein.

Gemäß einer besonders bevorzugten Ausführungsform ist das Barbitursäurederivat aus der Gruppe ausgewählt, die aus 1-Cyclohexyl-5-ethyl-barbitursäure, 1-Phenyl-5-ethyl-barbitursäure und 1,3,5-Trimethyl-barbitursäure besteht.

Der Anteil des wenigstens einen Barbitursäurederivats (a3) an der Paste A liegt vorzugsweise im Bereich von 0,1 - 10 Gewichtsprozent, mehr bevorzugt im Bereich von 0,5 - 8 Gewichtsprozent und noch mehr bevorzugt im Bereich von 1 - 5 Gewichtsprozent, bezogen auf das Gesamtgewicht der in Paste A enthaltenen Bestandteile.

Die Paste A ist vorzugsweise im Wesentlichen frei von Schwermetallverbindungen. Schwermetallverbindung in diesem Sinne sind Verbindungen von Metallen, die eine Dichte bei einer Temperatur von 20°C von wenigstens 3,5, vorzugsweise von wenigstens 5 aufweisen. Der Anteil an Schwermetallverbindungen in Paste A beträgt vorzugsweise weniger als 50 ppm, mehr bevorzugt weniger als 25 ppm, noch mehr bevorzugt weniger als 10 ppm und ganz besonders bevorzugt weniger als 5 ppm.

Paste B enthält wenigstens ein polymerisierbares Monomer (b1) mit einem pH-Wert in Wasser im Bereich von 5 - 9.

Das polymerisierbare Monomer (b1) ist bei einer Temperatur von 25°C und einem Druck von 1013 hPa vorzugsweise flüssig.

Das polymerisierbare Monomer (b1) ist vorzugsweise ein Methacrylsäureester. Vorzugsweise handelt es sich bei dem Methacrylsäureester (b1) um einen monofunktionellen Methacrylsäureester. Dieser ist vorzugsweise hydrophob. Durch die Verwendung von hydrophoben monofunktionellen Methacrylsäureestern (b1) kann eine spätere Volumenvergrößerung des Knochenzements infolge von Wasseraufnahme und damit eine Beschädigung des Knochens verhindert werden. Gemäß einer bevorzugten Ausführungsform ist der monofunktionelle Methacrylsäureester hydrophob, wenn er neben der Estergruppe keine weiteren polaren Gruppen enthält. Vorzugsweise weist der monofunktionelle, hydrophobe Methacrylsäureester keine Carboxylgruppen, Hydroxylgruppen, Amidgruppen, Sulfonsäuregruppen, Sulfatgruppen, Phosphatgruppen oder Phosphonatgruppen auf.

Bei den Estern handelt es sich vorzugsweise um Alkylester. Unter Alkylester fallen erfindungsgemäß auch Cycloalkylester. Die Alkylester sind gemäß einer bevorzugten Ausführungsform Ester von Methacrylsäure mit Alkoholen, die 1 - 20 Kohlenstoffatome, mehr bevorzugt 1 - 10 Kohlenstoffatome, noch mehr bevorzugt 1 - 6 Kohlenstoffatome und ganz besonders bevorzugt 1 - 4 Kohlenstoffatome aufweisen. Die Alkohole können substituiert oder nicht substituiert sein und sind vorzugsweise nicht substituiert. Die Alkohole können ferner gesättigt oder ungesättigt sein und sind vorzugsweise gesättigt.

Gemäß einer besonders bevorzugten Ausführungsform handelt es sich bei dem polymerisierbaren Monomer (b1) um Methacrylsäuremethylester und Methacrylsäureethylester.

Gemäß einer weiteren bevorzugten Ausführungsform handelt es sich bei dem polymerisierbaren Monomer (b1) nicht um einen von Bisphenol A abgeleiteten Methacrylsäureester.

Das erfindungsgemäß eingesetzte polymerisierbare Monomer (b1) weist vorzugsweise eine Molmasse von weniger als 1000 g/mol auf. Davon umfasst sind auch polymerisierbare Monomere, die Bestandteil einer Monomermischung sind, wobei wenigstens eines der polymerisierbaren Monomere der Monomermischung eine definierte Struktur mit einer Molmasse von weniger als 1000 g/mol aufweist.

Das polymerisierbare Monomer (b1) zeichnet sich dadurch aus, dass eine wässrige Lösung des polymerisierbaren Monomers (b1) einen pH-Wert im Bereich von 5 - 9, vorzugsweise im Bereich von 5,5 - 8,5, noch mehr bevorzugt im Bereich von 6 - 8 und ganz besonders bevorzugt im Bereich von 6,5 - 7,5 aufweist.

Paste B enthält vorzugsweise 15 - 85 Gewichtsprozent, mehr bevorzugt 20 - 70 Gewichtsprozent, noch mehr bevorzugt 25 - 60 Gewichtsprozent und besonders bevorzugt 25 - 50 Gewichtsprozent wenigstens eines polymerisierbaren Monomers (b1), bezogen auf das Gesamtgewicht der in Paste B enthaltenen Bestandteile. Demnach kann Paste B ein oder mehrere strukturell verschiedene polymerisierbare Monomere (b1) enthalten.

Paste B enthält ferner wenigstens einen in (b1) unlöslichen Füllstoff (b2).

Bei dem Füllstoff (b2) handelt es sich um einen bei Raumtemperatur festen Stoff, der in der Lage ist, die Viskosität der aus den übrigen in Paste B enthaltenen Bestandteilen zusammengesetzten Mischung zu erhöhen. Der Füllstoff (b2) soll biokompatibel sein.

Gemäß einer bevorzugten Ausführungsform ist der Füllstoff (b2) aus Polymeren, anorganischen Salzen, anorganischen Oxiden, Metallen und Metalllegierungen ausgewählt.

Der Füllstoff (b2) ist vorzugsweise partikulär. Gemäß einer besonders bevorzugten Ausführungsform weist der Füllstoff (b2) eine durchschnittliche Teilchengröße im Bereich von 10 nm - 100 µm und besonders bevorzugt im Bereich von 100 nm - 10 µm auf. Unter durchschnittlicher Teilchengröße wird vorliegend ein Größenbereich verstanden, der von wenigstens 90 Prozent der Teilchen eingenommen wird.

Im Rahmen der Erfindung werden unter dem Begriff Polymere sowohl Homopolymere als auch Copolymere zusammengefasst.

Bei dem als Füllstoff verwendbaren Polymer handelt es sich vorzugsweise um ein Polymer mit einer gewichtsmittleren Molmasse von wenigstens 150.000 g/mol. Beispielsweise kann es sich bei dem Polymer um ein Polymer oder Copolymer eines Methacrylsäureesters handeln. Gemäß einer besonders bevorzugten Ausführungsform ist das wenigstens eine Polymer aus der Gruppe ausgewählt, die aus Polymethacrylsäuremethylester (PMMA), Polymethacrylsäureethylester (PMAE), Polymethacrylsäurepropylester (PMAP), Polymethacrylsäureisopropylester, Poly(methylmethacrylat-co-methylacrylat) und Poly(styren-co-methylmethacrylat) besteht. Das Polymer kann jedoch ebenso aus der Gruppe ausgewählt sein, die aus Polyethylen, Polypropylen oder Polybutadien besteht. Das Polymer kann zudem vernetzt oder unvernetzt sein.

Das als Füllstoff (b2) verwendbare anorganische Salz kann ein im polymeriserbaren Monomer lösliches oder unlösliches Salz sein. Vorzugsweise handelt es sich bei dem anorganischen Salz um ein Salz eines Elements, das aus der 2. Hauptgruppe des Periodensystems der Elemente ausgewählt wird. Gemäß einer bevorzugten Ausführungsform ist das anorganische Salz ein Salz von Calcium, Strontium oder Barium. Gemäß einer besonders bevorzugten Ausführungsform ist das anorganische Salz Calciumsulfat, Bariumsulfat oder Calciumcarbonat.

Bei dem als Füllstoff (b2) verwendbaren anorganischen Oxid kann es sich vorzugsweise um ein Metalloxid handeln. Gemäß einer bevorzugten Ausführungsform ist das anorganische Oxid ein Oxid der Übergangsmetalle. Gemäß einer besonders bevorzugten Ausführungsform handelt es sich bei dem anorganischen Oxid um Titandioxid oder Zirkoniumdioxid.

Bei dem als Füllstoff (b2) verwendbaren Metall kann es sich beispielsweise um ein Übergangsmetall handeln. Gemäß einer bevorzugten Ausführungsform ist das Metall Tantal oder Wolfram.

Die als Füllstoff (b2) verwendbare Metalllegierung ist eine Legierung von wenigstens zwei Metallen. Vorzugsweise enthält die Legierung wenigstens ein Übergangsmetall. Gemäß einer besonders bevorzugten Ausführungsform weist die Legierung wenigstens Tantal oder Wolfram auf. Bei der Legierung kann es sich auch um eine Legierung aus Tantal und Wolfram handeln.

Der Füllstoff (b2) ist in dem polymerisierbaren Monomer (b1) mit einem pH-Wert im Bereich von 5 - 9 (b1) unlöslich. Erfindungsgemäß ist der Füllstoff (b2) in dem polymerisierbaren Monomer mit einem pH-Wert im Bereich von 5 - 9 (b1) unlöslich, wenn der Füllstoff (b2) bei einer Temperatur von 25°C eine Löslichkeit im polymerisierbaren Monomer (b1) von weniger als 50 g/l, vorzugsweise weniger als 25 g/l, mehr bevorzugt weniger als 10 g/l und noch mehr bevorzugt weniger als 5 g/l aufweist.

Der Anteil des wenigstens einen Füllstoffs (b2) an der Paste B beträgt vorzugsweise weniger als 85 Gewichtsprozent, mehr bevorzugt weniger als 80 Gewichtsprozent und noch mehr bevorzugt weniger als 75 Gewichtsprozent, bezogen auf das Gesamtgewicht der in Paste B enthaltenen Bestandteile. Vorzugsweise enthält Paste B 15 - 85 Gewichtsprozent, mehr bevorzugt 15 - 80 Gewichtsprozent und noch mehr bevorzugt 20 - 75 Gewichtsprozent an dem wenigstens einen Füllstoff (b2), bezogen auf das Gesamtgewicht der in Paste B enthaltenen Bestandteile.

Paste B enthält außerdem wenigstens ein in dem polymerisierbaren Monomer (b1) lösliches Peroxid (b3).

Unter einem Peroxid (b3) werden erfindungsgemäß Verbindungen verstanden, die wenigstens eine Peroxogruppe (-O-O-)enthalten.

Das Peroxid (b3) weist vorzugsweise keine freien aciden Gruppen auf.

Bei dem Peroxid (b3) kann es sich um ein anorganisches Peroxid, wie zum Beispiel ein toxikologisch unbedenkliches Hydroperoxid, oder ein organisches Peroxid handeln.

Erfindungsgemäß ist das Peroxid (b3) in dem polymerisierbaren Monomer (b1) löslich, wenn sich das Peroxid (b3) in dem polymerisierbaren Monomer (a1) zu wenigstens 1 g/l, vorzugsweise zu wenigstens 3 g/l, noch mehr bevorzugt zu wenigstens 5 g/l und ganz besonders bevorzugt zu wenigstens 10 g/l bei einer Temperatur von 25°C löst.

Gemäß einer besonders bevorzugten Ausführungsform ist das Peroxid (b3) aus der Gruppe ausgewählt, die aus Dibenzoylperoxid und Dilauroylperoxid besteht.

Paste B enthält vorzugsweise 0,01 - 12 Gewichtsprozent, vorzugsweise 0,03 - 10 Gewichtsprozent, mehr bevorzugt 0,05 - 8 Gewichtsprozent und noch mehr bevorzugt 0,1 - 5 Gewichtsprozent wenigstens eines Peroxids (b3), bezogen auf das Gesamtgewicht der in Paste B enthaltenen Bestandteile.

Die Paste B weist ferner wenigstens eine in (b1) unlösliche Schwermetallverbindung (b4) auf, die aus der Gruppe ausgewählt ist, die aus Schwermetallsalzen und Schwermetallkomplexen besteht.

Unter Schwermetallverbindung werden erfindungsgemäß Verbindungen von Metallen verstanden, die eine Dichte bei einer Temperatur von 20°C von wenigstens 3,5, vorzugsweise von wenigstens 5 aufweisen.

Gemäß einer bevorzugten Ausführungsform handelt es sich bei der in (b1) unlöslichen Schwermetallverbindung (b4) um eine basische Schwermetallverbindung. Unter basischer Schwermetallverbindung wird eine Schwermetallverbindung verstanden, die in Wasser gelöst oder suspendiert einen pH-Wert von wenigstens 6,5, bevorzugt wenigstens 7 und noch mehr bevorzugt wenigstens 7,5 aufweist.

Gemäß einer ganz besonders bevorzugten Ausführungsform handelt es sich bei den Schwermetallverbindungen um Verbindungen von Metallen, die ihre Oxidationsstufe wechseln können.

Erfindungsgemäß bevorzugt sind Verbindungen von Kupfer (II), Eisen (II), Eisen (III), Mangan (II), Mangan (III), Cobalt (II) und Cobalt (III).

Die erfindungsgemäßen Schwermetallverbindungen sind in der Lage, sich in Gegenwart und der Barbitursäurederivate (a3) in eine in dem polymerisierbaren Monomer (a1) bzw. (a2) lösliche Form umzuwandeln.

Erfindungsgemäß handelt es sich bei den Schwermetallverbindungen um Schwermetallsalze oder Schwermetallkomplexe.

Bei den Schwermetallsalzen handelt es sich vorzugsweise um Halogenide, Hydroxyde, Carbonate oder Carbonsäuresalze von Schwermetallen. Bevorzugte Schwermetallsalze sind Salze von Kupfer (II), Eisen (II), Eisen (III), Mangan (II), Mangan (III), Cobalt (II) und Cobalt (III).

Gemäß einer Ausführungsform wird das Schwermetallsalz aus der Gruppe ausgewählt, die aus Kupferhydroxid, Cobalt(II)-hydroxid und basischem Kupfercarbonat besteht.

Gemäß einer besonders bevorzugten Ausführungsform handelt es sich bei der in (b1) unlöslichen Schwermetallverbindung (b4) um ein Halogenidsalz. Nach dieser Ausführungsform kann das Erfordernis, wonach in wenigstens einer der Pasten A und B ein Halogenidsalz enthalten ist, durch das die unlösliche Schwermetallverbindung darstellende Halogenidsalz erfüllt sein. Folglich kann erfindungsgemäß ein Halogenidsalz zugleich die in (b1) unlösliche Schwermetallverbindung als auch das als Halogenidionen-Donator darstellende Halogenidsalz von Paste B repräsentieren. Dementsprechend kann es sich bei dem in Paste B enthaltenen Halogenidsalz und bei der in (b1) unlöslichen Schwermetallverbindung (b4) um dieselbe Verbindung handeln. Das Halogenidsalz kann vorzugsweise aus der Gruppe ausgewählt sein, die aus Chloriden und Bromiden von Schwermetallen besteht. Gemäß einer besonders bevorzugten Ausführungsform handelt es sich bei dem Halogenidsalz um eine Verbindung, die aus der Gruppe ausgewählt ist, die aus Kupfer(II)-chlorid, Mangan(II)-chlorid, Eisen(II)-chlorid, Eisen(III)-chlorid, Kobalt(II)-chlorid und Kobalt(III)-chlorid besteht.

Der Anteil der Schwermetallverbindung (b4) an Paste B liegt vorzugsweise im Bereich von 0,0005 - 0,5 Gewichtsprozent, mehr bevorzugt im Bereich von 0,001 - 0,05 Gewichtsprozent und ganz besonders bevorzugt im Bereich von 0,001 - 0,01 Gewichtsprozent, bezogen auf das Gewicht von Paste B.
In wenigstens einer der der Pasten A und B ist ferner wenigstens ein Halogenidionen-Donator enthalten.

Der Halogenidionen-Donator ist ein Halogenidsalz. Bei dem Halogenidsalz kann es sich beispielsweise um ein anorganisches Halogenidsalz oder ein organisches Halogenidsalz handeln. Das Halogenidsalz ist in diesen Fällen vorzugsweise ein Salz von Chlor oder Brom.
Gemäß einer besonders bevorzugten Ausführungsform ist das Halogenidsalz ein quartäres Ammoniumsalz eines Halogenids. Das quartäre Ammoniumsalz kann vorzugsweise ein quartäres Alkyl-, Aryl-, Aryldialkyl-, Diarylalkyl- oder Cycloalkyldialkylammoniumsalz sein. Ein bevorzugtes Halogenidsalz ist beispielsweise Trioctylmethylammoniumchlorid.
Gemäß einer weiteren besonders Ausführungsform kann es sich bei dem Halogenidsalz auch um ein Hydrohalogenid, vorzugsweise ein Hydrochlorid oder Hydrobromid, handeln. Das Halogenidsalz kann dabei vorzugsweise ein Hydrohalogenid einer tertiären Ammoniumverbindung sein.
Gemäß noch einer weiteren bevorzugten Ausführungsform kann das Halogenidsalz ein Halogenid mit einem Metallkation oder einem Metalloxidkation sein. Als Metallkation kommen beispielsweise die Kationen von Lithium (Li⁺), Zink (Zn²⁺) und Zirkonium (Zr⁴⁺) in Betracht. Ein bevorzugtes Metalloxidkation kann zum Beispiel das Zirkoniumoxidkation, ZrO²⁺, sein. Vorzugsweise handelt es sich bei dem Halogenidsalz mit einem Metallkation oder einem Metalloxidkation um ein Halogenidsalz, das aus der Gruppe ausgewählt ist, die aus Lithiumchlorid (LiCI), Zinkdichlorid (ZnCl₂) und Zirkoniumoxiddichlorid (ZrOCl₂) besteht.
Der Halogenidionen-Donator weist gemäß einer weiteren bevorzugten Ausführungsform eine Löslichkeit von wenigstens 10 g/l, mehr bevorzugt von wenigstens 25 g/l, noch mehr bevorzugt von wenigstens 50 g/l und noch mehr bevorzugt von wenigstens 100 g/l, in dem polymerisierbaren Monomer (a1) und/oder (b1), das in der Paste, in der auch der Halogenidionen-Donator enthalten ist, auf.

Gemäß einer besonders bevorzugten Ausführungsform ist es auch möglich, dass das Halogenidsalz in Paste B enthalten ist und es sich hierbei um die in (b1) unlösliche Schwermetallverbindung (b4) handelt. Dementsprechend kann es sich bei dem in Paste B enthaltenen Halogenidsalz und bei der in (b1) unlöslichen Schwermetallverbindung (b4) um dieselbe Verbindung handeln. Gemäß dieser Ausführungsform kann es bevorzugt sein, dass das Halogenidsalz aus der Gruppe ausgewählt ist, die aus Chloriden und Bromiden von Schwermetallen besteht. Gemäß einer besonders bevorzugten Ausführungsform handelt es sich bei dem Halogenidsalz um eine Verbindung, die aus der Gruppe ausgewählt ist, die aus Kupfer(II)-chlorid, Mangan(II)-chlorid, Eisen(II)-chlorid, Eisen(III)-chlorid, Kobalt(II)-chlorid und Kobalt(III)-chlorid besteht.

Der Anteil des Halogenidionen-Donators an der jeweiligen Paste beträgt vorzugsweise 0,005 - 10 Gewichtsprozent, mehr bevorzugt 0,001 - 8 Gewichtsprozent, noch mehr bevorzugt 0,01 - 5 Gewichtsprozent und ganz besonders bevorzugt 0,3 - 3 Gewichtsprozent, bezogen auf das Gewicht der Paste, in der der Halogenidionen-Donator enthalten ist.

Handelt es sich bei der wenigstens einen als Halogenidionen-Donator fungierenden Verbindung und der wenigstens einen in (b1) unlöslichen Schwermetallverbindung (b4) um dasselbe Halogenidsalz oder dieselben Halogenidsalze, so kann es bevorzugt sein, dass der Anteil dieses Halogenidsalzes bzw. dieser Halogenidsalze an Paste B im Bereich von 0,005 - 10 Gewichtsprozent, mehr bevorzugt im Bereich von 0,001 - 8 Gewichtsprozent, noch mehr bevorzugt im Bereich von 0,01 - 5 Gewichtsprozent und ganz besonders bevorzugt im Bereich von 0,001 - 0,01 Gewichtsprozent, bezogen auf das Gewicht von Paste B liegt.

Die Pasten des erfindungsgemäßen Kits können ferner weitere Bestandteile aufweisen.

In wenigstens einer der Pasten kann beispielsweise wenigstens ein Haftvermittler enthalten sein. Dieser Haftvermittler begünstigt das Haftvermögen des Knochenzementes auf der Prothese. Als geeigneter Haftvermittler hat sich beispielsweise Methacrylamid erwiesen.

Ferner kann wenigstens eine der Pasten wenigstens einen Vernetzer aufweisen. Bei dem Vernetzer handelt es sich vorzugsweise um eine bifunktionelle oder trifunktionelle Verbindung. Erfindungsgemäß soll der Vernetzer eine Vernetzung des bei der Aushärtung des Knochenzementes polymerisierenden Monomeren bewirken. Gemäß einer bevorzugten Ausführungsform weist der Vernetzer wenigstens zwei (Meth-)acrylatgruppen auf. Besonders bevorzugt ist der Vernetzer aus der Gruppe ausgewählt, die aus Ethylenglycoldimethacrylat, Butylenglycoldimethacrylat (z.B. Butan-1,4-diol-dimethacrylat) und Hexamethylendimethacrylat (z.B. Hexan-1,6-diol-dimethacrylat) besteht.

Erfindungsgemäß kann wenigstens eine der Pasten auch wenigstens einen Röntgenopaker aufweisen. Der Röntgenopaker ist vorzugsweise aus der Gruppe ausgewählt, die aus Metalloxiden (wie zum Beispiel Zirkoniumdioxid), Bariumsulfat, toxikologisch unbedenkliche Schwermetallpartikel (wie zum Beispiel Tantal), Ferrit und Magnetit (ggf. auch supramagnetisches Magnetit) besteht. Diese Röntgenopaker weisen vorzugsweise einen mittleren Teilchendurchmesser im Bereich von 10 nm - 500 µm auf. Daneben kann es auch zweckmäßig sein, im polymerisierbaren Monomer lösliche röntgenopake Verbindungen zu verwenden. Beispielhaft sind dafür Ester der 3,5-Bis(acetamido)-2,4,6-triiodbenzoesäure. Ebenfalls ist es möglich, Gadolinium-Verbindungen, wie Gadolinium-Chelat mit den Estern der 1,4,7,10-Tetraazacyclododecan-1,4,7,10-tetraessigsäure (DOTA), als Kontrastmittel für die Magnetresonanztomographie in wenigstens eine der Pasten zu integrieren.

Gemäß einer bevorzugten Ausführungsform enthält wenigstens eine der Pasten, vorzugsweise aber beide Pasten, ein in dem polymerisierbaren Monomer, das in dieser Paste enthalten ist, wenigstens ein lösliches Polymer. Dieses Polymer ist erfindungsgemäß in dem polymerisierbaren Monomer, das in der Paste enthalten ist, in der auch das lösliche Polymer enthalten ist, löslich, wenn sich das Polymer in diesem polymerisierbaren Monomer zu wenigstens 10 g/l, vorzugsweise zu wenigstens 25 g/l, mehr bevorzugt zu wenigstens 50 g/l und ganz besonders bevorzugt zu wenigstens 100 g/l, löst. Das in dem polymerisierbaren Monomer lösliche Polymer kann ein Homopolymer oder ein Copolymer sein. Bei diesem Polymer handelt es sich vorzugsweise um ein Polymer mit einer gewichtsmittleren Molmasse von wenigstens 150.000 g/mol. Beispielsweise kann es sich bei dem Polymer um ein Polymer oder Copolymer eines Methacrylsäureesters handeln. Gemäß einer besonders bevorzugten Ausführungsform ist das wenigstens eine Polymer aus der Gruppe ausgewählt, die aus Polymethacrylsäuremethylester (PMMA), Polymethacrylsäureethylester (PMAE), Polymethacrylsäurepropylester (PMAP), Polymethacrylsäureisopropylester, Poly(methylmethacrylat-co-methylacrylat) und Poly(styren-co-methylmethacrylat) besteht. Der Anteil des in dem polymerisierbaren Monomer löslichen Polymers an der Paste, die dieses Polymer enthält, liegt vorzugsweise im Bereich von 5 - 50 Gewichtsprozent, mehr bevorzugt im Bereich von 10 - 40 Gewichtsprozent und ganz besonders bevorzugt im Bereich von 20 - 30 Gewichtsprozent.

Wenigstens eine der Pasten kann außerdem wenigstens einen Stabilisator aufweisen. Der Stabilisator soll geeignet sein, eine Spontanpolymerisation der in den Pasten enthaltenen Monomere zu verhindern. Ferner soll der Stabilisator keine störenden Wechselwirkungen mit den anderen in den Pasten enthaltenen Bestandteilen zeigen. Derartige Stabilisatoren sind aus dem Stand der Technik bekannt. Gemäß einer bevorzugten Ausführungsform handelt es sich bei dem Stabilisator um 2,6-Di-tert-butyl-4-methylphenol und/oder 2,6-Di-tert-butyl-phenol.

Wenigstens eine der Pasten kann ferner wenigstens eine pharmazeutische Substanz enthalten. Gemäß einer besonders bevorzugten Ausführungsform ist diese pharmazeutische Substanz in Paste A enthalten. Andererseits kann die pharmazeutische Substanz auch oder lediglich in Paste B enthalten sein, sofern sie eine hinreichend hohe Stabilität gegenüber den in Paste B enthaltenen Bestandteilen aufweist. Die wenigstens eine pharmazeutische Substanz kann in Paste A und/oder Paste B in gelöster oder suspendierter Form enthalten sein.

Die pharmazeutische Substanz kann vorzugsweise aus der Gruppe ausgewählt sein, die aus Antibiotika, Aniphlogistika, Steroide, Hormonen, Wachstumsfaktoren, Bisphosphonaten, Cytostatika und Genvektoren besteht. Gemäß einer besonders bevorzugten Ausführungsform handelt es sich bei der wenigstens einen pharmazeutischen Substanz um ein Antibiotikum.

Das wenigstens eine Antibiotikum wird vorzugsweise ausgewählt aus den Gruppen der Aminoglykosid-Antibiotika, der Glykopeptid-Antibiotika, der Lincosamid-Antibiotika, der Gyrasehemmer, der Carbapeneme, der cyclischen Lipopeptide, der Glycylcycline, der Oxazolidone und der Polypeptidantibiotika.

Gemäß einer besonders bevorzugten Ausführungsform handelt es sich bei dem wenigstens einen Antibiotikum um ein Mitglied, das aus der Gruppe ausgewählt ist, die aus Gentamicin, Tobramycin, Amikacin, Vancomycin, Teicoplanin, Dalbavancin, Lincosamin, Clindamycin, Moxifloxacin, Levofloxacin, Ofloxacin, Ciprofloxacin, Doripenem, Meropenem, Tigecyclin, Linezolid, Eperezolid, Ramoplanin, Metronidazol, Tinidazol, Omidazol und Colistin sowie Salzen und Estern davon besteht.

Demnach kann das wenigstens eine Antibiotikum aus der Gruppe ausgewählt sein, die aus Gentamicinsulfat, Gentamicinhydrochlorid, Amikacinsulfat, Amikacinhydrochlorid, Tobramycinsulfat, Tobramycinhydrochlorid, Clindamycinhydrochlorid, Lincosaminhydrochlorid und Moxifloxacin besteht.

Das wenigstens eine Antiphlogistikum ist vorzugsweise aus der Gruppe ausgewählt, die aus nicht-steroidalen Antiphlogistika und Glukokortikoiden besteht. Gemäß einer besonders bevorzugten Ausführungsform ist das wenigstens eine Antiphlogistikum aus der Gruppe ausgewählt, die aus Acetylsalicylsäure, Ibuprofen, Diclofenac, Ketoprofen, Dexamethason, Prednison, Hydrocortison, Hydrocortisonacetat und Fluticason besteht.

Das wenigstens eine Hormon ist vorzugsweise aus der Gruppe ausgewählt, die aus Serotonin, Somatotropin, Testosteron und Östrogen besteht.

Der wenigstens eine Wachstumsfaktor ist vorzugsweise aus der Gruppe ausgewählt, die aus dem Fibroblast Growth Factor (FGF), dem Transforming Growth Factor (TGF), dem Platelet Derived Growth Factor (PDGF), dem Epidermalen Wachstumsfaktor (EGF), dem Vascular Endothelial Growth Factor (VEGF), insulinähnlichen Wachstumsfaktoren (IGF), dem Hepatocyte Growth Factor (HGF), dem Bone Morphogenetic Protein (BMP), Interleukin-1B, Interleukin 8 und dem Nervenwachstumsfaktor besteht.

Das wenigstens eine Cytostatikum ist vorzugsweise aus der Gruppe ausgewählt, die aus Alkylantien, Platinanaloga, Interkalantien, Mitosehemmern, Taxanen, Topoisomerasehemmern und Antimetaboliten besteht.

Das wenigstens eine Bisphosphonat ist vorzugsweise aus der Gruppe ausgewählt, die aus Zoledronat und Aledronat besteht.

Wenigstens eine der Pasten kann ferner wenigstens einen Farbstoff aufweisen. Bei dem Farbstoff kann es sich besonders bevorzugt um einen Lebensmittelfarbstoff handeln. Gemäß einer besonders bevorzugten Ausführungsform wird der Farbstoff aus der Gruppe ausgewählt, die aus E101, E104, E132, E141 (Chlorophyllin), E142, Riboflavin und Lissamingrün besteht. Unter den Begriff Farbstoff fallen erfindungsgemäß auch Farblacke, wie zum Beispiel Farblack Grün, das Aluminiumsalz einer Mischung aus E104 und E132.

Gemäß einer besonders bevorzugten Ausführungsform enthält Paste A wenigstens 20 Gewichtsprozent des polymerisierbaren Monomers (a1), wenigstens 20 Gewichtsprozent des unlöslichen Füllstoffs (a2), wenigstens 0,1 Gewichtsprozent des Barbitursäurederivats (a3) und wenigstens 0,05 Gewichtsprozent des Halogenidionen-Donators und Paste B wenigstens 20 Gewichtsprozent des polymerisierbaren Monomers (b1), wenigstens 20 Gewichtsprozent des unlöslichen Füllstoffs (b2), wenigstens 0,01 Gewichtsprozent des löslichen Peroxids (b3), wenigstens 0,01 Gewichtsprozent der Schwermetallverbindung (b4) und wenigstens 0,05 Gewichtsprozent des Halogenidionen-Donators.

Gemäß einer weiteren besonders bevorzugten Ausführungsform enthält Paste A 15 - 85 Gewichtsprozent des polymerisierbaren Monomers (a1), 15 - 85 Gewichtsprozent des unlöslichen Füllstoffs (a2), 0,1 - 10 Gewichtsprozent des Barbitursäurederivats (a3) und 0,05 - 10 Gewichtsprozent des Halogenidionen-Donators und Paste B 15 - 85 Gewichtsprozent des polymerisierbaren Monomers (b1), 15 - 85 Gewichtsprozent des unlöslichen Füllstoffs (b2), 0,01 - 12 Gewichtsprozent des löslichen Peroxids (b3), 0,001 - 0,05 Gewichtsprozent der Schwermetallverbindung (b4) und 0,05 - 10 Gewichtsprozent des Halogenidionen-Donators.

Erfindungsgemäß dient der Kit, der wenigstens die Pasten A und B enthält, zur Herstellung von Knochenzement.

Dazu werden die wenigstens zwei Pasten A und B miteinander vermischt, wobei wiederum eine Paste, Paste C, erhalten wird.

Das Mischungsverhältnis beträgt vorzugsweise 0,5 - 1,5 Gewichtsteile an Paste A und 0,5 - 1,5 Gewichtsteile an Paste B. Gemäß einer besonders bevorzugten Ausführungsform beträgt der Anteil der Paste A 30 - 70 Gewichtsprozent und der Anteil der Paste B 30 - 70 Gewichtsprozent, bezogen auf das Gesamtgewicht der Pasten A und B.

Das Mischen kann mit üblichen Mischgeräten, beispielsweise einem statischen Mischer oder einem dynamischen Mischer erfolgen.

Das Mischen kann unter Vakuum erfolgen. Die Verwendung des erfindungsgemäßen Initiatorsystems erlaubt jedoch auch das vakuumfreie Mischen der Paste A und B ohne Beeinträchtigung der Eigenschaften des Knochenzements.

Nach dem Vermischen der Pasten des Kits ist die letztlich erhaltene Paste C nach der Norm ISO 5833 klebfrei und kann sofort verarbeitet werden.
Der aus der Paste C durch Aushärtung entstehende Knochenzement erreicht etwa sechs bis acht Minuten nach dem Vermischen der im Kit enthaltenen Pasten eine hohe Festigkeit.

Der erfindungsgemäße Kit kann gemäß einer bevorzugten Ausführungsform zur Herstellung einer Paste zur mechanischen Fixierung von Gelenkendoprothesen, zur Herstellung einer Paste zur Abdeckung von Schädeldefekten, zur Herstellung einer Paste zur Auffüllung von Knochenkavitäten, zur Herstellung einer Paste zur Femuroplastie, zur Herstellung einer Paste zur Vertebroplastie, zur Herstellung einer Paste zur Kyphoplastie, zur Herstellung einer Paste zur Herstellung von Spacern und zur Herstellung einer Paste zur Herstellung von Trägermaterialien für die lokale Antibiotika-Therapie verwendet werden.
Unter dem Begriff "Spacer" werden dabei erfindungsgemäß Implantate verstanden, die temporär im Rahmen des zweizeitigen Prothesenwechsels bei septischen Revisionen als Platzhalter verwendet werden.
Trägermaterialien für die lokale Antibiotika-Therapie können als Kugeln oder kugelähnliche Körper oder als bohnenförmige Körper ausgebildet sein. Daneben ist es auch möglich, stabförmige oder scheibenförmige Trägermaterialien herzustellen, die den aus dem erfindungsgemäßen Kit hergestellten Knochenzement hergestellten Knochenzement erhalten. Weiterhin können die Trägermaterialien auch auf resorbierbarem oder nicht resorbierbarem Fadenmaterial perlschnurförmig aufgereiht sein.
Die vorstehend beschriebenen erfindungsgemäßen Verwendungen von Knochenzement sind aus der Literatur bekannt und dort mannigfach beschrieben.
Erfindungsgemäß wird der Kit für die vorstehend beschrieben Verwendungen eingesetzt, indem vorzugsweise aus den im Kit enthaltenen Pasten durch Vermischen eine Paste hergestellt wird, die analog zu den aus dem Stand der Technik bekannten Pasten für die beschriebenen Verwendungen eingesetzt wird.
Die Erfindung wird durch die nachstehend beschriebenen Beispiel erläutert, die jedoch die Erfindung nicht einschränken.

### Beispiele:

In den Beispielen wurden verschiedene Kits zur Herstellung von Knochenzement bereitgestellt, zur Herstellung von Knochenzement verwendet und die Eigenschaften des erhaltenen Knochenzements verglichen.
Beispiel 1 betrifft einen erfindungsgemäßen Kit mit den beiden Pasten A und B.
Beispiel 2 betrifft einen erfindungsgemäßen Kit mit den beiden Pasten A und B, der zusätzlich mit Gentamicinsulfat ein Antibiotikum enthält.

Vergleichsbeispiel 1 betrifft einen aus der DE 10 2007 050 762 B3 bekannten Kit mit den beiden Pasten A und B.

Vergleichsbeispiel 2 betrifft einen kommerziell erhältlichen Kit, Palacos® R, mit einer Flüssigkomponente, die das zu polymerisierende Monomer enthält, und einer Feststoffkomponente, die als Füllstoff ein Polymer enthält.

Vergleichsbeispiel 3 betrifft einen kommerziell erhältlichen Kit, Palacos® R+G, mit einer Flüssigkomponente, die das zu polymerisierende Monomer enthält, und einer Feststoffkomponente, die als Füllstoff ein Polymer enthält. Palacos® R+G unterscheidet sich von Palacos® R durch die zusätzliche Gegenwart des Antibiotikums Gentamicinsulfat.

### a) Bereitstellung der Kits:

Zur Bereitstellung der Kits der Beispiele 1 und 2 sowie Vergleichsbeispiel 1 wurden die jeweiligen Pasten A und B durch intensive Vermischung der jeweiligen Komponenten (wie in Tabelle 1 angegeben) hergestellt. Die Kits der Vergleichsbeispiele 2 (Palacos® R; Charge 7034) und 3 (Palacos® R+G; Charge 7047) wurden kommerziell erworben.

**Tabelle 1: Zusammensetzung der in den Kits der Beispiele 1 und 2 sowie in Vergleichsbeispiel 1 enthalten Pasten.**

| | **Beispiel 1** | | **Beispiel 2** | | **Vergleichsbeispiel 1** | |
|---|---|---|---|---|---|---|
| | **Paste** | | **Paste** | | **Paste** | |
| **Komponente** | **A** | **B** | **A** | **B** | **A** | **B** |
| Methylmethacrylat | 16,2 g | 16,2 g | 16,2 g | 16,2 g | 16,2 g | 16,2 g |
| Methacrylamid | 0,4 g | 0,4 g | 0,4 g | 0,4 g | 0,4 g | 0,4 g |
| Ethylenglykoldimethacrylat | 0,6 g | 0,6 g | 0,6 g | 0,6 g | - | - |
| Zirkoniumdioxid | 2,4 g | 2,4 g | 2,4 g | 2,4 g | 2,8 g | 4,0 g |
| Lösliches Poly(methylmethacrylat-comethylacrylat) | 10,0 g | 10,0 g | 10,0 g | 10,0 g | 10,6 g | 10,8 g |
| Nicht lösliches Polymethylmethacrylat | 9,0 g | 9,0 g | 8,0 g | 9,0 g | 8,4 g | 8,6 g |
| 2,6-Di-t-butyl-4-methylphenol | 20 mg | - | 20 mg | - | 20 mg | 20 mg |
| Aliquat 336 (Trioctylmethylammoniumchlorid) | 250 mg | 250 mg | 250 mg | 250 mg | - | - |
| 1-Cyclohexyl-5-ethyl-barbiturat | 1,5 g | - | 1,5 g | - | 1,6 g | - |
| Kupfer(II)-hydroxid | - | 1,0 mg | - | 1,0 mg | - | - |
| Kupfer(II)-2-ethylhexanoat | - | - | - | - | - | 1 mg |
| Dibenzoylperoxid | - | 0,5 g | - | 0,5 g | - | - |
| Gentamicinsulfat | - | - | 1,0 g | - | - | - |
| Summarischer Schwermetallgehalt | < 10 ppm | | < 10 ppm | | nicht bestimmt | |

### b) Herstellung von Knochenzement unter Verwendung der Kits

In den Beispielen 1 und 2 sowie Vergleichsbeispiel 1 wurden die zum jeweiligen Kit gehörenden Pasten A und B im Gewichtsverhältnis 1:1 intensiv miteinander vermischt. Die erhaltenen Mischungen wurden anschließend in rechteckige Formen mit einer Höhe von 3,3 mm eingebracht, wo sie bis zur Aushärtung stehen gelassen wurden.

In den Vergleichsbeispielen 2 und 3 wurden die zum jeweiligen Kit gehörenden Feststoffkomponenten und Flüssigkomponenten unter Verwendung eines Vakuum-Mischgerätes intensiv miteinander vermischt. Die erhaltenen Mischungen wurden anschließend in rechteckige Formen mit einer Höhe von 3,3 mm eingebracht, wo sie bis zur Aushärtung stehen gelassen wurden.

### c) Eigenschaften des erhaltenen Knochenzements

Für die Prüfung der 4-Punkt-Biegefestigkeit und des Biegemoduls wurden nach erfolgter Aushärtung aus den erhaltenen Zementplatten Streifen mit einer Länge von 75 mm und einer Breite von 10 mm gesägt.

Die Prüfung der 4-Punkt-Biegung und des Biegemoduls erfolgte nach 24 Stunden Lagerung der Prüfkörper in Luft bei 23 °C mit einer Zwick-Universal-Prüfmaschine.

Für die Prüfung der Druckfestigkeit wurden zylindrische Prüfkörper mit einer Höhe von 12 mm und einem Durchmesser von 6 mm angefertigt. Die Messung erfolgte ebenfalls nach 24 Stunden Lagerung bei 23 °C an der Luft unter Verwendung einer Zwick-Universal-Prüfmaschine.

Die Ergebnisse dieser Prüfungen sind in Tabelle 2 dargestellt.

**Tabelle 2: Ergebnisse der Prüfungen zur 4-Punkt-Biegefestigkeit und zum Biegemodul der erhaltenen Knochenzemente nach 24 h Lagerung an Luft.**

| **Zement** | **4-Punkt-Biegefestigkeit [MPa]** | **Biegemodul [MPa]** |
|---|---|---|
| Beispiel 1 | 70,5 ± 1,7 | 2730 ± 17 |
| Beispiel 2 | 57,5 ± 2,8 | 2350 ± 66 |
| Vergleichsbeispiel 1 | 47,0 ± 1,5 | 1809 ± 81 |
| Vergleichsbeispiel 2 | 68,6 ± 0,7 | 2925 ± 50 |
| Vergleichsbeispiel 3 | 69,0 ±1,3 | 2945 ± 52 |

Weiterhin wurden Probekörper unmittelbar nach ihrer Herstellung 48 Stunden in Wasser bei 37 °C gelagert. Im Anschluss erfolgte die Bestimmung der 4-Punktbiegefestigkeit und des Biegemoduls wie vorstehend beschrieben. Die Ergebnisse dieser Prüfungen sind in Tabelle 3 angegeben.

**Tabelle 3: Ergebnisse der Prüfungen zur 4-Punkt-Biegefestigkeit und zum Biegemodul der erhaltenen Knochenzemente nach 48 h Lagerung in Wasser.**

| **Zement** | **4-Punkt-Biegefestigkeit [MPa]** | **Biegemodul [MPa]** |
|---|---|---|
| Beispiel 1 | 79,6 ± 5,2 | 3035 ± 74 |
| Beispiel 2 | 69,4 ± 3,4 | 2726 ± 58 |
| Vergleichsbeispiel 1 | 68,6 ± 1,8 | 2577 ± 94 |
| Vergleichsbeispiel 2 | 74,0 ± 1,3 | 2882 ± 46 |
| Vergleichsbeispiel 3 | 66,5 ± 1,4 | 2774 ± 39 |

Wie aus Tabelle 2 ersichtlich ist, weisen die unter Verwendung des erfindungsgemäßen Kits hergestellten Knochenzemente der Beispiele 1 und 2 gegenüber dem Knochenzement, der aus dem Stand der Technik bekannt ist und ebenfalls auf dem Einsatz zweier Pasten beruht (Vergleichsbeispiel 1), sowohl eine deutlich erhöhte 4-Punkt-Biegefestigkeit als auch ein deutlich erhöhtes Biegemodul nach 24 h Lagerung auf. Dies zeigt die stark erhöhte initiale Festigkeit des erfindungsgemäßen Pastensystems. Insbesondere zeigen die unter Verwendung des erfindungsgemäßen Kits hergestellten Knochenzemente der Beispiele 1 und 2 Werte für eine 4-Punkt-Biegefestigkeit und ein Biegemodul nach 24 h Lagerung, die mit denen von herkömmlichen Kits aus einer Feststoffkomponente und einer Flüssigkomponente vergleichbar sind, ohne dass jedoch die mit letztgenannten Systemen verbundenen Nachteile (z.B. aufwändige Vakuum-Mischsysteme, giftige Monomerdämpfe) vorherrschen.

Tabelle 3 zeigt, dass sich die Festigkeit des erhaltenen Knochenzements während der Lagerung in Wasser, das letztlich die Transplantationsumgebung nachahmen soll, bei den aus dem Stand der Technik bekannten Systemen gemäß Vergleichsbeispiel 1 nachteiligerweise sprunghaft erhöht im Vergleich zu den erfindungsgemäßen Systemen und den bekannten Systemen aus Feststoffkomponente und Flüssigkomponente.

In Tabelle 4 ist das Verhältnis der Werte für die 4-Punkt-Biegefestigkeit und das Biegemodul des Knochenzements nach 24 h Lagerung an Luft zu den Werten für die 4-Punkt-Biegefestigkeit und Biegemodul nach 48 h Lagerung in Wasser angegeben (initiale 4-Punkt-Biegefestigkeit und initiales Biegemodul).

**Tabelle 4: Initiale 4-Punkt-Biegefestigkeit und initiales Biegemodul der in den Beispielen 1 und 2 sowie Vergleichsbeispiel 1 erhaltenen Knochenzemente.**

| **Zement** | **Initiale 4-Punkt-Biegefestigkeit [MPa]** | **Initiales Biegemodul [MPa]** |
|---|---|---|
| Beispiel 1 | 88,6 % | 90,0 % |
| Beispiel 2 | 82,9 % | 86,2 % |
| Vergleichsbeispiel 1 | 68,5 % | 66,4 % |

Tabelle 4 zeigt, dass sich bei dem mittels des erfindungsgemäßen Kits hergestellten Knochenzement die 4-Punkt-Biegefestigkeit und das Biegemodul vorteilhafterweise erheblich früher einstellen als beim aus dem Stand der Technik bekannten Knochenzement.

## Patentansprüche

1. Kit aufweisend eine Paste A und eine Paste B, wobei
(a) Paste A
(a1) ein polymerisierbares Monomer mit einem pH-Wert in Wasser im Bereich von 5 - 9,
(a2) einen in (a1) unlöslichen Füllstoff und
(a3) ein Barbitursäurederivat, das aus der Gruppe ausgewählt ist, die aus 1,5-disubstituierten Barbituraten, 1,3,5-trisubtituierten Barbituraten und 1,3,5-tetrasubtituierten Barbituraten besteht, enthält, und
(b) Paste B
(b1) ein polymerisierbares Monomer mit einem pH-Wert in Wasser im Bereich von 5 - 9,
(b2) einen in (b1) unlöslichen Füllstoff,
(b3) ein in (b1) lösliches Peroxid,
(b4) eine in (b1) unlösliche Schwermetallverbindung, die aus der Gruppe ausgewählt ist, die aus Schwermetallsalzen und Schwermetallkomplexen besteht, enthält,
wobei in wenigstens einer der Pasten A und B ein Halogenidsalz enthalten ist.

2. Kit nach Anspruch 1, **dadurch gekennzeichnet, dass** das polymerisierbare Monomer in Paste A ein Methacrylat-Monomer ist.

3. Kit nach Anspruch 1 oder 2, **dadurch gekennzeichnet, dass** Paste A ein in (a1) lösliches Polymer enthält.

4. Kit nach einem der Ansprüche 1 - 3, **dadurch gekennzeichnet, dass** der unlösliche Füllstoff (a2) ein partikuläres Polymer ist.

5. Kit nach einem der vorangegangenen Ansprüche, **dadurch gekennzeichnet, dass** das polymerisierbare Monomer in Paste B ein Methacrylat-Monomer ist.

6. Kit nach einem der vorangegangenen Ansprüche, **dadurch gekennzeichnet, dass** Paste B ein in (b1) lösliches Polymer enthält.

7. Kit nach einem der vorangegangenen Ansprüche, **dadurch gekennzeichnet, dass** der unlösliche Füllstoff (b2) ein partikuläres Polymer ist.

8. Kit nach einem der vorangegangenen Ansprüche, **dadurch gekennzeichnet, dass** die in (b1) unlösliche Schwermetallverbindung (b4) eine basische Schwermetallverbindung ist.

9. Kit nach einem der vorangegangenen Ansprüche, **dadurch gekennzeichnet, dass** das Halogenidsalz in Paste B enthalten ist und es sich hierbei um die in (b1) unlösliche Schwermetallverbindung (b4) handelt.

10. Verwendung eines Kits nach einem der Ansprüche 1 - 8 zur Herstellung einer Paste zur mechanischen Fixierung von Gelenkendoprothesen, zur Herstellung einer Paste zur Abdeckung von Schädeldefekten, zur Herstellung einer Paste zur Auffüllung von Knochenkavitäten, zur Herstellung einer Paste für die Femuroplastie, zur Herstellung einer Paste für die Vertebroplaste, zur Herstellung einer Paste für die Kyphoplastie, zur Herstellung einer Paste zur Herstellung von Spacern oder zur Herstellung einer Paste zur Herstellung von Trägermaterialien für die lokale Antibiotika-Therapie.

## Claims

1. Kit comprising a paste A and a paste B, whereby
(a) paste A contains
(a1) a polymerisable monomer with a pH value in water in the range of 5 - 9;
(a2) a filling agent that is insoluble in (a1); and
(a3) a barbituric acid derivative selected from the group consisting of 1, 5-disubstituted barbiturates, 1,3,5-trisubstituted barbiturates, and 1,3,5-tetrasubstituted barbiturates; and
(b) paste B contains
(b1) a polymerisable monomer with a pH value in water in the range of 5 - 9;
(b2) a filling agent that is insoluble in (b1);
(b3) a peroxide that is soluble in (b1);
(b4) a heavy metal compound that is insoluble in (b1) and is selected from the group consisting of heavy metal salts and heavy metal complexes; whereby at least one of the pastes A and B contains a halide salt.

2. Kit according to claim 1, **characterised in that** the polymerisable monomer in paste A is a methacrylate monomer.

3. Kit according to claim 1 or 2, **characterised in that** paste A contains a polymer that is soluble in (a1).

4. Kit according to any one of the claims 1 - 3, **characterised in that** the insoluble filling agent (a2) is a particulate polymer.

5. Kit according to any one of the preceding claims, **characterised in that** the polymerisable monomer in paste B is a methacrylate monomer.

6. Kit according to any one of the preceding claims, **characterised in that** paste B contains a polymer that is soluble in (b1).

7. Kit according to any one of the preceding claims, **characterised in that** the insoluble filling agent (b2) is a particulate polymer.

8. Kit according to any one of the preceding claims, **characterised in that** the heavy metal compound (b4) that is insoluble in (b1) is an alkaline heavy metal compound.

9. Kit according to any one of the preceding claims, **characterised in that** paste B contains the halide salt and **in that** the halide salt is the heavy metal compound (b4) that is insoluble in (b1).

10. Use of a kit according to any one of the claims 1 - 8 for the production of a paste for mechanical fixation of articular prostheses, for the production of a paste for coverage of skull defects, for the production of a paste for the filling of bone cavities, for the production of a paste for femuroplasty, for the production of a paste for vertebroplasty, for the production of a paste for kyphoplasty, for the production of a paste for the production of spacers or for the production of a paste for the production of carrier materials for local antibiotics therapy.

## Revendications

1. Kit présentant une pâte A et une pâte B,
(a) la pâte A contenant
(a1) un monomère polymérisable avec un taux de pH dans l'eau de l'ordre de 5 à 9,
(a2) une matière de remplissage insoluble dans (a1) et
(a3) un dérivé de l'acide barbiturique qui est sélectionné dans le groupe se composant de barbituriques disubstitués en 1,5, de barbituriques trisubstitués en 1,3,5 et de barbituriques tétrasubstitués en 1,3,5, et
(b) la pâte B contenant
(b1) un monomère polymérisable avec un taux de pH dans l'eau de l'ordre de 5 à 9,
(b2) une matière de remplissage insoluble dans (b1),
(b3) un peroxyde soluble dans (b1),
(b4) un composé de métaux lourds insoluble dans (b1), qui est sélectionné dans le groupe se composant de sels de métaux lourds et de complexes de métaux lourds,
un sel d'halogénure étant contenu au moins dans l'une des pâtes A et B.

2. Kit conformément à la revendication n°1, **caractérisé par le fait que** le monomère polymérisable dans la pâte A est un monomère de méthacrylate.

3. Kit conformément à la revendication n°1 ou n°2, **caractérisé par le fait que** la pâte A contient un polymère soluble dans (a1).

4. Kit conformément à l'une des revendications n°1 à n°3, **caractérisé par le fait que** la matière de remplissage (a2) insoluble est un polymère particulaire.

5. Kit conformément à l'une des revendications précédentes, **caractérisé par le fait que** le monomère polymérisable dans la pâte B est un monomère de méthacrylate.

6. Kit conformément à l'une des revendications précédentes, **caractérisé par le fait que** la pâte B contient un polymère soluble dans (b1).

7. Kit conformément à l'une des revendications précédentes, **caractérisé par le fait que** la matière de remplissage (b2) insoluble est un polymère particulaire.

8. Kit conformément à l'une des revendications précédentes, **caractérisé par le fait que** le composé de métaux lourds (b4) insoluble dans (b1) est un composé de métaux lourds basique.

9. Kit conformément à l'une des revendications précédentes, **caractérisé par le fait que** le sel d'halogénure est contenu dans la pâte B et qu'il s'agit du composé de métaux lourds (b4) insoluble dans (b1).

10. Utilisation d'un kit conformément à l'une des revendications n°1 à n°8 pour la fabrication d'une pâte pour la fixation mécanique d'endoprothèses articulaires, pour la fabrication d'une pâte pour masquer des défauts crâniens, pour la fabrication d'une pâte pour combler des cavités osseuses, pour la fabrication d'une pâte pour la fémoroplastie, pour la fabrication d'une pâte pour la vertébroplastie, pour la fabrication d'une pâte pour la kyphoplastie, pour la fabrication d'une pâte pour la fabrication d'espaceurs ou pour la fabrication d'une pâte pour la fabrication de matériaux de support pour le traitement antibiotique local.
